# EUROPÄISCHE PATENTSCHRIFT

(11) **EP 3 923 913 B1**
(45) Veröffentlichungstag und Bekanntmachung des Hinweises auf die Patenterteilung: **07.06.2023**
(21) Anmeldenummer: 21701948.8
(22) Anmeldetag: 18.01.2021
(51) Int. Cl.: A61L 9/015

(54) **NATURSTOFFPAD**
NATURAL MATERIAL PAD
TAMPON DE MATÉRIAU NATUREL

(30) Priorität: 29.04.2020 DE 102020111668
(43) Veröffentlichungstag der Anmeldung: 22.12.2021
(73) Patentinhaber: Organoid Technologies GmbH, 6500 Fließ (AT)
(72) Erfinder: JEHART, Martin, 6473 Wenns (AT); EGGER, Christoph, 6020 Innsbruck (AT)
(74) Vertreter: Frick, Robert
(86) Internationale Anmeldenummer: PCT/EP2021/050950
(87) Internationale Veröffentlichungsnummer: WO 2021/219255

(56) Entgegenhaltungen:
- WO-A1-2012/063225
- CN-A- 110 090 285
- DE-A1-102014 113 770
- DE-A1-102017 110 405

## Beschreibung

Die Erfindung betrifft ein Naturstoffpad mit einer Naturstoffmatrix, die mit Wirk- und/oder Duftstoffen beladen ist, sowie ein Verfahren zu dessen Herstellung.

Im medizinischen, heilpraktischen und kosmetischen Bereich kommen Wirkstoffe und Wirkstoffgemische zum Einsatz, die durch beispielsweise Extraktion aus Pflanzen gewonnen werden. Bekannte Darreichungsformen für derartige Wirkstoffe bzw. Wirkstoffgemische umfassen Cremes, Salben, Lotionen, ätherische Öle, Tropfen, Tinkturen und Wickel zum Auftragen auf die Haut bzw. auf Schleimhäute oder Darreichungsformen in Form von Tabletten, Pastillen, Tropfen und Säften zur oralen Einnahme. Auch eine Raumbeduftung mit ätherischen Pflanzenölen zur Erzielung einer beruhigenden oder stimulierenden Wirkung oder einfach wegen des Geruchs ist in den letzten Jahren immer populärer geworden.

Im Stand der Technik sind auch unterschiedliche Arten von Wirkstoffpflastern bekannt geworden, wie sie beispielsweise in der WO 91/09592 A1 beschrieben sind. Bekannte Beispiele umfassen Nikotinpflaster, aber es gibt zahlreiche andere Beispiele. Auch in der Medizin, Kosmetik und Naturheilkunde werden Pflaster und Pads zum Auftrag auf die Haut bereits zahlreich verwendet.

Ein Nachteil bekannter Verabreichungsformen von ätherischen Ölen und anderen Formen pflanzlicher Wirkstoffgemische, unter anderem auch bekannter Pflaster und Pads ist eine fehlende optische und haptische Korrelation der Verabreichungsform mit der Pflanze bzw. den Pflanzen, aus der der verabreichte Wirkstoff bzw. die verabreichten Wirkstoffe gewonnen werden. Obwohl Pflanzen auf der Verpackung abgebildet werden können und auch der Geruch der Medikamente, Kosmetika und Naturheilmittel auf den Ursprung schließen lässt, könnten optische und haptische Aspekte das Behandlungserlebnis und den Wertigkeitseindruck beim Verbraucher weiter steigern.

Aufgabe der Erfindung ist es, eine Bereitstellungsform für pflanzliche Wirk- und/oder Duftstoffe bereitzustellen, welche bekannten Bereitstellungsformen in optischer und haptischer Hinsicht überlegen sind.

Vor diesem Hintergrund betrifft die Erfindung ein Naturstoffpad in Form eines Schichtsystems mit einer flächigen Naturstoffmatrix, die auf einem Träger angeordnet und von einem Überzug überdeckt ist, wobei die Naturstoffmatrix ein vorzugsweise zerkleinertes Pflanzenmaterial, ein Bindemittel und am Pflanzenmaterial gehaltene oder anderweitig in der Naturstoffmatrix verteilte Wirk- und/oder Duftstoffe umfasst. Der Träger und der Überzug dienen als Barriere- bzw. Sperrschicht für Wirk- und/oder Duftstoffe der Naturstoffmatrix, wie sie später noch näher beschrieben sind.

Das erfindungsgemäße Naturstoffpad hat eine flächige Gestalt und die Naturstoffmatrix ist sandwichartig zwischen Träger und Überzug eingeschlossen.

In einer Ausführungsform wird der Überzug durch zusätzliches Bindemittel gebildet. Das Bindemittel des Überzugs entspricht vorzugsweise dem auch in der Naturstoffmatrix verwendeten Bindemittel, sodass die Naturstoffmatrix und der Überzug nahtlos ineinander übergehen.

Alternativ kann in einer anderen Ausführungsform vorgesehen sein, dass der Überzug durch eine Folie gebildet wird.

In einer Ausführungsform handelt es sich bei dem Naturstoffpad um ein selbstklebendes Naturstoffpad. In diesem Fall weist vorzugsweise der Träger zumindest an seiner von der Naturstoffmatrix abgewandten Unterseite eine Klebeoberfläche auf, anhand welcher das Naturstoffpad an seiner exponierten Unterseite auf eine Oberfläche geklebt werden kann.

Bei dem Träger handelt es sich in dieser Ausführungsform vorzugsweise um eine zumindest einseitig klebend ausgeführte Kunststofffolie, es kann sich aber in anderen Varianten der Erfindung auch um ein zumindest einseitig klebend ausgeführtes Papier handeln. Im Falle einer Kunststofffolie wird vorzugsweise eine Folie verwendet, die biologisch abbaubar ist. Ferner kommen vorzugsweise ein hautverträglicher Kunststoff sowie ein dermatologisch getesteter Klebstoff zum Einsatz.

In einer anderen Ausführungsform, vornehmlich in Varianten des Naturstoffpads, die nicht selbstklebend ausgebildet sind, kann als Träger eine netzartige Struktur verwendet werden. Die netzartige Struktur kann in einer Ausführungsform aus Pflanzenfasern bestehen. Beispiele umfassen Gewebe oder Vliese aus Baumwolle, Hanf, Flachs, Kenaf, Heu oder Lavendelstängel. Alternativ kann die netzartige Struktur teilweise oder vollständig aus synthetischen Fasern wie beispielsweise Polyamid-Fasern gefertigt sein.

In einer Ausführungsform weisen der Träger und/oder der Überzug eine Vielzahl an Öffnungen auf, durch welche Wirk- und/oder Duftstoffe der Naturstoffmatrix abgegeben werden können. Während auch Materialien für den Träger und den Überzug verwendet werden können, durch die flüchtige Wirk- bzw. Duftstoffe diffundieren können, sind Träger und Überzug in der Regel zumindest so undurchlässig für diese flüchtigen Bestandteile, dass eine kontrollierte Abgabe durch Öffnungen in Träger oder Überzug erreicht wird. Öffnungen im Träger sind insbesondere dann sinnvoll, wenn die Wirkstoffe auf der Unterseite des Naturstoffpads abgegeben werden sollen.

Dies ist beispielsweise dann der Fall, wenn das Naturstoffpad selbstklebend ausgeführt ist und als Wirkstoffpflaster verwendet werden soll, dessen Wirkstoffe durch die Haut aufgenommen werden sollen. Öffnungen im Überzug sind dagegen dann sinnvoll, wenn die Wirkstoffe auf der Oberseite des Naturstoffpads abgegeben werden sollen. Dies ist beispielsweise dann der Fall, wenn das Naturstoffpad als Aufkleber für Gebrauchsgegenstände oder als Wirkstoff- und/oder Duftreservoir für Räume verwendet werden soll, dessen Wirkstoffe an die Umgebungsluft abgegeben werden sollen.

Vorzugsweise sind entweder der Träger oder der Überzug mit Öffnungen versehen, aber nicht sowohl die Deckschicht als auch die Klebeschicht. In einer anderen Variante, insbesondere einer nicht selbstklebenden Variante zur Abgabe von Wirk- oder Duftstoffen an Räume, können aber auch sowohl der Träger als auch der Überzug Öffnungen aufweisen.

Die Öffnungen können durch Perforation des Trägers oder Überzugs gebildet werden, beispielsweise durch Perforation anhand einer Stachelwalze. In einer anderen Variante können Öffnungen im Überzug dadurch gebildet sein, dass ein aus zusätzlichem Bindemittel gebildeter Überzug nur dünn aufgetragen ist und Erhebungen in der Naturstoffmatrix durch den Überzug ragen, sodass die Benetzung nicht vollständig ist. In einer wiederum anderen Variante können Öffnungen im Träger durch originär im Träger vorhandene Öffnungen gebildet sein, was beispielsweise bei netzartigen oder perforierten Strukturen eine Rolle spielen kann.

Anhand des erfindungsgemäßen Naturstoffpads können pflanzliche Wirkstoffe und Wirkstoffkombinationen, die aus der Medizin und Naturheilkunde an sich bekannt sind, in stimmiger und ansprechender Weise bereitgestellt werden. Das Naturstoffpad ist vielseitig einsetzbar. Selbstklebende Varianten können beispielsweise als Hautpflaster zur Wirkstoffabgabe an die Haut oder an die Umgebung, oder als Aufkleber für Oberflächen unterschiedlicher Gegenstände, von Mobiltelefonen bis hin zu Möbelstücken und Innenwänden von Häusern zum Einsatz kommen. Weitere Anwendungen umfassen die Aufbringung des Naturstoffpads auf Gesichtsmasken, wobei zur Desinfektion der eingeatmeten Luft die Naturstoffpads beispielsweise mit Kampferöl oder Pfefferminzöl beladen sein können. Nicht selbstklebende Varianten können beispielsweise als Tapeten zum Einsatz kommen, als Duftanhänger oder als Bestandteil von Gebrauchsgegenständen wie Vorhängen oder Lampenschirmen, als Therapiehilfsmittel und Spielzeug zum Erkennen von Düften in Kombination mit Haptik, zur Anbringung an Kleidungsstücke, beispielsweise als Einnähpad, zur auswechselbaren Anbringung mit etwa einer Klettverbindung, oder als Einlegegegenstand für die Tasche eines Kleidungsstücks, als Akustikpaneel, auf Geschenkverpackungen, auf Handycovers, als Accessoire oder als Werbeartikel.

Der Überzug ist vorzugsweise an der Oberfläche nicht glatt, sondern folgt den Konturen des Pflanzenmaterials in der Naturstoffmatrix. So kann ein haptischer Eindruck entstehen, bei dem der Benutzer auch eine durch die Naturmaterialien gegebene Struktur wahrnehmen kann, die beispielsweise bei Fichtennadeln oder Grashalmen ausgeprägt sein kann.

Vorzugsweise ist das erfindungsgemäße Naturstoffpad an der die Öffnungen aufweisenden Seite mit einem abziehbaren Schutzfilm versehen. Der Schutzfilm ist im Wesentlichen undurchlässig für die Wirk- und/oder Duftstoffe des Naturstoffpads. Er dient als Siegel und verhindert deren ungewollte Freisetzung. Das Naturstoffpad wird im Vertrieb und Handel typischerweise mit Schutzfilm bereitgestellt. Der Benutzer kann den Schutzfilm dann abziehen, wenn er das Naturstoffpad in Benutzung nehmen möchte. Bei dem Schutzfilm handelt es sich in der Regel um einen Kunststofffilm oder ein silikoniertes bzw. antihaftbeschichtetes Papier.

Alternativ zu einem Schutzfilm kann die mit Öffnungen versehene und insbesondere perforierte Seite des Naturstoffpads auch mit einem abreibbaren Überzug, beispielsweise aus Xanthan oder einem anderen Geliermittel, versiegelt sein. Die Aktivierung des Naturstoffpads kann dann durch Abreiben oder eine andere mechanische Aktivierung erfolgen.

Weiterhin kann auch die Verwendung von Schutzüberzügen auf dem Träger vorgesehen sein, die sich durch UV-Licht oder Feuchtigkeitskontakt zersetzen bzw. auflösen.

Auch kann die Oberfläche des Naturstoffpads so ausgeführt sein, dass es durch eine besondere Mischung des Pflanzenmaterials und der Wirk- und/oder Duftstoffe mit dem Bindemittel zu einer geringen und langandauernden Emission der Wirk- und/oder Duftstoffe kommt, ohne dass eine weitere Öffnung oder Aktivierung stattfinden muss.

Das Bindemittel fixiert das Pflanzenmaterial und vereinigt es zu einer stabilen Matrix. Insbesondere eignen sich als Bindemittel solche, welche die folgenden Substanzen enthalten, alleine oder in Kombination: Harnstoffleim, Naturharze, Methylzellulose, Stärke, Polylactide, Naturkautschuk, Polyvinylacetat, Polyurethan oder Cyanacrylat.

Die Wirk- und/oder Duftstoffe können insbesondere pflanzlichen Ursprungs sein und weiter vorzugsweise in flüssiger Form vorliegen. Die Flüssigkeit kann am Pflanzenmaterial oder einem zusätzlichen Trägermaterial innerhalb der Naturstoffmatrix gebunden sein. Es kann sich um ein Pflanzenextrakt handeln, aber auch jede andere Form von Flüssigkeiten oder Stoffen pflanzlichen Ursprungs, die beispielsweise durch Pressen oder dergleichen gewonnen werden, können eingesetzt werden, also alle Arten von Auszügen, Lösungen, Extrakten, Essenzen und dergleichen. Die Wirk- und/oder Duftstoffe umfassen vorzugsweise ätherische Öle bzw. eine Mischung von pflanzlichen Wirkstoffen. Sie können als Lösung vorliegen, wobei es sich bei dem Lösungsmittel in unterschiedlichen Ausführungsformen der Erfindung um ein organisches Lösungsmittel oder um ein wässriges Lösungsmittel handeln kann. Die folgenden Komponenten können in geeigneten Lösungsmitten vorhanden sein: Ethanol und andere Alkohole, Ketone, Terpene, Aldehyde, Ester und Phenole. Besonders bevorzugt sind Lösungsmittel auf Basis einer Mischung von Wasser und einem Alkohol, da in einem derartigen Lösungsmittel einerseits organische Stoffe gut gelöst werden können, da das Naturstoffpad aber andererseits keine gegebenenfalls schädlichen Lösungsmitteldämpfe emittiert.

In einer Variante der Erfindung können die Wirk- und/oder Duftstoffe als flüssiges Konzentrat vorliegen. Unter einem Konzentrat ist eine aufkonzentrierte Lösung zu verstehen, die aus einer verdünnten Lösung, beispielsweise einer Extraktionslösung durch Verringerung des Lösungsmittelvolumens gewonnen wird.

In einer anderen Variante der Erfindung können die Wirk- und/oder Duftstoffe als pures Pflanzenöl oder als O/W- oder W/O-Emulsion vorliegen.

Die Flüssigkeit der Wirk- und/oder Duftstoffe kann an dem Pflanzenmaterial adsorbiert oder im Pflanzenmaterial absorbiert oder beides sein. Alternativ oder zusätzlich kann in der Naturstoffmatrix ein Träger vorhanden sein, das mit der Flüssigkeit beladen ist. Bei dem zusätzlichen Trägermaterial kann es sich um ein pulver- oder teilchenförmiges Material wie beispielsweise Zeolithmaterial, oder um ein textiles Material handeln. Der Einsatz eines zusätzlichen Trägers kann insbesondere dann vorgesehen sein, wenn das Pflanzenmaterial selbst nur wenig Aufnahmekapazität für die Flüssigkeit aufweist.

Alternativ können die Wirk- und/oder Duftstoffe auch in fester Form, beispielsweise als pulver- oder teilchenförmiges Material (in Form von beispielsweise Granulaten, Schnitzeln oder Spänen) in die Naturstoffmatrix eingebracht sein.

Als Pflanzenmaterial, welches vorzugsweise zerkleinert bzw. zerschnitten in der Naturstoffmatrix vorliegen soll, kann beispielsweise Material zum Einsatz kommen, das auf Moose oder Flechten zurückgeht. Derartige Materialien haben unter natürlichen Pflanzenmaterialien die hier sehr gewinnbringende Eigenschaft einer hohen Aufnahme- und Speicherkapazität für Flüssigkeiten.

In einer Ausführungsform kann vorgesehen sein, dass das Pflanzenmaterial aus einer einzigen Pflanzenfamilie, vorzugsweise einer einzigen Pflanzengattung und weiter vorzugsweise einer einzigen Pflanzenart gefertigt ist. Alternativ oder zusätzlich kann vorgesehen sein, dass die Wirk- und/oder Duftstoffe auf eine einzige Pflanzenfamilie, vorzugsweise eine einzige Pflanzengattung und weiter vorzugsweise eine einzige Pflanzenart zurückgehen. Das Naturstoffpad kann in dieser Ausführungsform die Wirkstoffe einer einzigen Pflanze aufweisen.

In einer anderen Ausführungsform kann vorgesehen sein, dass das Pflanzenmaterial sich aus Materialien unterschiedlicher Pflanzenarten, Pflanzengattungen oder gar Pflanzenfamilien zusammensetzt. Alternativ oder zusätzlich können die Wirk- und/oder Duftstoffe auf unterschiedliche Pflanzenarten, Pflanzengattungen oder gar Pflanzenfamilien zurückgehen. In der Naturheilkunde kommen auch vielfach Wirkstoffkombinationen aus unterschiedlichen Pflanzen zum Einsatz. Dieses Konzept wird in der vorliegenden Ausführungsform auf das erfindungsgemäße Naturstoffpad übertragen.

Im Rahmen der Erfindung ist bevorzugt, dass das Pflanzenmaterial und die Wirk- und/oder Duftstoffe zumindest teilweise auf dieselbe Pflanzenfamilie, vorzugsweise dieselbe Pflanzengattung und weiter vorzugsweise dieselbe Pflanzenart zurückgehen. Wenn sowohl das Pflanzenmaterial als auch die Wirk- und/oder Duftstoffe auf dieselbe Pflanzenfamilie, vorzugsweise dieselbe Pflanzengattung und weiter vorzugsweise sogar dieselbe Pflanzenart zurückgehen führt dies zu einer stimmigen Kombination aus korrelierenden Trägermaterialien und Wirkstoffen.

In einer Ausführungsform ist vorgesehen, dass die Naturstoffmatrix mehrschichtig aufgebaut ist. Mehrere Schichten können unterschiedliche Pflanzenmaterialien enthalten, also Materialien unterschiedlicher Pflanzen oder unterschiedliche Materialien derselben Pflanze (auch andere Schnitte oder Faserlängen). In einem Beispiel kann etwa eine Grundschicht aus abgesiebten Heublumen mit Stücklängen von 1 mm bis 3 mm, eine Mittellage aus saugfähigen Moos mit Stücklängen von 5 mm bis 15 mm und eine Decklage besteht aus vereinzelt aufgestreuten Rosenblüten mit geringem Deckungsgrad in der Naturstoffmatrix enthalten sein.

Neben den Pflanzenmaterialien und dem Binder kann die Naturstoffmatrix ferner auch Stoffe nicht biogenen Ursprungs wie beispielsweise Zeolithgranulat, Füllstoffe oder Speicherstoffe enthalten.

Das Pflanzenmaterial der Naturstoffmatrix ist vorzugsweise nicht dasjenige Pflanzenmaterial, aus dem die die Wirk- und/oder Duftstoffe gewonnen sind. So umfasst auch das Pflanzenmaterial der Naturstoffmatrix selbst noch Wirk- und/oder Duftstoffe. Alternativ kann aber auch dasjenige Pflanzenmaterial für die Naturstoffmatrix verwendet werden, aus dem zuvor die Wirk- und/oder Duftstoffe gewonnen wurden.

Die Dosierung der Abgabegeschwindigkeit bzw. die Abgabedauer der Wirkstoffe aus dem erfindungsgemäßen Naturstoffpad kann durch die Anordnung, Lochgröße, Fläche und Anzahl der Öffnungen und durch das Mischungsverhältnis der Naturstoffe zu Bindemittel bestimmt werden.

In einer Ausführungsform kann vorgesehen sein, dass die Naturstoffmatrix eine Substanz umfasst, die bei Luftkontakt austrocknet und so die Öffnungen verschließt. Beispiele umfassen Geliermittel wie etwa Xanthan. Die Öffnungen werden somit nach einer Erstaktivierung des Naturstoffpads später wieder verschlossen und die Inhaltsstoffe einstweilen konserviert. Erst durch mechanische Belastung der so versiegelten Oberfläche, etwa durch Reiben mit der Hand, kann die Versiegelung wieder entfernt und die Funktion wieder hergestellt werden. So kann das Naturstoffpad immer wieder aktiviert werden, wenn Bedarf besteht, und verbraucht sich wesentlich langsamer.

Das erfindungsgemäße Naturstoffpad kann in vorkonfektionierter Größe oder in Form einer größeren Einheit bereitgestellt werden, von der ein Benutzer Teile einer gewünschten Größe portionieren kann, beispielsweise abschneiden, ausstanzen oder auf eine andere Weise abtrennen kann. Das Naturstoffpad kann in einer luftdichten Verpackung bereitgestellt werden, um ein ungewolltes seitliches Entweichen der Wirk- bzw. Duftstoffe während längerer Lagerzeiten zu verhindern.

Die Gesamtdicke des Naturstoffpads kann beispielsweise zwischen 0,1 mm und 10 mm, vorzugsweise zwischen 0,2 mm und 4 mm liegen und beträgt weiter vorzugsweise zwischen 1 mm und 2 mm. Mindestens 30%, vorzugsweise mindestens 50% und weiter vorzugsweise mindestens 70% der Dicke können dabei in bevorzugten Varianten der Erfindung von der Naturstoffmatrix beansprucht werden.

Die Erfindung betrifft weiterhin ein Verfahren zur Herstellung eines erfindungsgemäßen Naturstoffpads mit den folgenden Schritten: a) Bereitstellen des Trägers; b) Positionieren des Trägers auf einer Unterlage; c) Auftragen des Pflanzenmaterials und des Bindemittels auf den Träger, um die Naturstoffmatrix auszubilden; und d) Ausbildung des Überzugs. Die Wirk- und/oder Duftstoffe werden entweder vor Ausbildung des Überzugs, beispielsweise gemeinsam mit dem Pflanzenmaterial in Schritt c), oder nachträglich durch Öffnungen im Träger oder Überzug in die Naturstoffmatrix eingebracht.

In einer bevorzugten Ausführungsform erfolgt die Ausbildung des Überzugs in Schritt d) durch das Auftragen von weiterem Bindemittel auf die Naturstoffmatrix.

Vorzugsweise kann das Verfahren einen weiteren Schritt e) zur Perforierung des Trägers oder des Überzugs anhand vorzugsweise einer Stachelwalze aufweisen, um Öffnungen in den Träger oder Überzug einzuarbeiten.

Das Pflanzenmaterial und das Bindemittel können in Schritt c) gemeinsam oder gestaffelt aufgetragen werden. Zudem kann in Schritt c) in einer Ausführungsform ein zusätzliches Trägermaterial für Wirk- und/oder Duftstoffe aufgetragen werden, ebenfalls entweder gemeinsam mit dem Pflanzenmaterial und/oder dem Trägermaterial oder gestaffelt.

Im Falle eines selbstklebenden Trägers kann dieser mit einem abziehbaren Schutzfilm an der klebend ausgeführten Seite bereitgestellt werden. Im Falle einer Perforierung des Trägers in einem optionalen Schritt e) wird vorzugsweise ein bestehender abziehbarer Schutzfilm ausgetauscht oder ein neuer abziehbarer Schutzfilm aufgebracht.

Generell kann in einem letzten Schritt ein abziehbarer Schutzfilm auf die die Öffnungen aufweisende Seite des Naturstoffpads aufgebracht werden.

Schritt c) kann gegebenenfalls auch unter Erwärmen auf eine Temperatur von größer 40°C, insbesondere größer 100°C erfolgen. Auch eine sequentielle Auftragung von vorzugsweise zerkleinertem Pflanzenmaterial und Bindemittel kann unter Schritt c) erfolgen.

Zwischen Schritten c) und d) kann eine Zwischentrocknung erfolgen, um eine formstabile Matrix zu bilden, bevor der Überzug ausgebildet wird. Die Zwischentrocknung kann gegebenenfalls unter Erwärmen auf eine Temperatur von größer 40°C, insbesondere größer 100°C erfolgen. Alternativ kann aber auch weiteres Bindemittel vor einer Härtung aufgetragen werden, sodass der Überzug gemeinsam mit der Matrix aushärtet, wodurch sich eine bessere Integration der Schichten ergibt.

Die Auftragung von Bindemittel in Schritten c) und/oder d) kann beispielsweise durch Aufgießen, Aufstreichen, Walzen, Sprühen oder durch Vorbeleimung des Pflanzenmaterials in einem Trommelmischer erfolgen. In einer Variante kann Sprühen bevorzugt sein.

Einzelheiten zu den Materialien des Trägers, des Überzugs, des Bindemittels, der Naturstoffmatrix und den Wirk- und/oder Duftstoffen können der obenstehenden Beschreibung des erfindungsgemäßen Naturstoffpads entnommen werden.

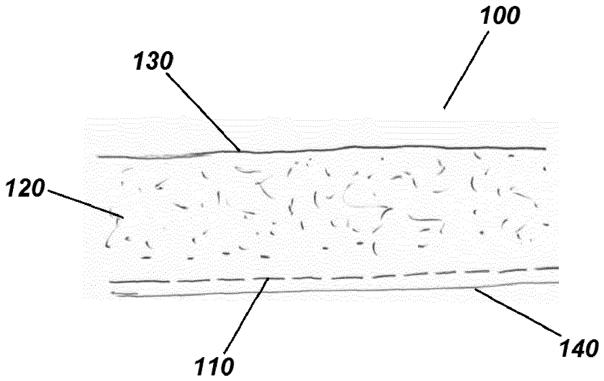

Weitere Einzelheiten und Vorteile der Erfindung ergeben sich aus den nachfolgend anhand der Figur beschriebenen Ausführungsbeispielen.

Die einzige Figur zeigt einen Querschnitt durch ein erfindungsgemäßes Naturstoffpad 100, bei dem es sich um ein selbstklebendes Schichtsystem mit einem Träger in Form einer Klebeschicht 110, einer Naturstoffmatrix 120 und einem Überzug 130 als Schichten handelt. Die Naturstoffmatrix 120 ist dabei als mittlere Schicht sandwichartig zwischen der Klebeschicht 110 und dem Überzug 130 eingeschlossen. Die Klebeschicht 110 und der Überzug 130 dienen als Barriere- bzw. Sperrschicht für Wirkstoffe der Naturstoffmatrix 120.

Die Klebeschicht 110 weist an ihrer von der Naturstoffmatrix 120 abgewandten Unterseite eine Haftschicht auf und ist dort von einem daran anhaftenden aber abziehbaren Abdeckpapier 140 überzogen.

Die Naturstoffmatrix 120 setzt sich aus zerkleinerten Pflanzenteilen und einem Bindemittel zusammen und ist mit einem flüssigen Pflanzenextrakt beladen.

Der Überzug 130 besteht aus einer durchgehenden Schicht desselben Bindemittels, das auch in der Naturstoffmatrix 120 zur Anwendung kommt. Er ist jedoch frei von Pflanzenteilen und auch nicht in nennenswertem Umfang mit dem flüssigen Pflanzenextrakt beladen.

Der Überzug 130 stellt eine Barriere für den Austritt flüchtiger Bestandteile des Pflanzenextrakts dar. Dasselbe gilt grundsätzlich für die Klebeschicht 110, wobei die Klebeschicht 110 durch eine definierte Perforation einen Austritt flüchtiger Bestandteile des Pflanzenextrakts mit gewisser Rate erlaubt.

Das Abdeckpapier 140 ist nicht perforiert. So kann ein Austritt flüchtiger Bestandteile des Pflanzenextrakts aus der Naturstoffmatrix 120 durch die Perforation der Klebeschicht 110 so lange verhindert werden, bis das Naturstoffpad 100 von einem Benutzer durch Abziehen des Abdeckpapiers 140 von der Klebeschicht 110 aktiviert wird.

Das Pflanzenextrakt wird durch Extraktion nach beispielsweise dem Soxhlet-Prinzip aus einem pflanzlichen Extraktionsgut gewonnen, das einen oder mehrere physiologisch aktive Wirkstoffe enthält. Dabei stehen Pflanzen mit einer in der Medizin, Kosmetik bzw. Naturkosmetik und Naturheilkunde erforschten Wirkung im Vordergrund bzw. auch Pflanzen, bei denen die medizinischen Wirkstoffe isoliert oder nachgebaut werden können und dann in Cremen oder Tabletten eingesetzt werden. Beispiele umfassen Moose oder Kräuter, beispielsweise Zahnkraut. Es kann sich um eine alkoholische oder wässrige Lösung, um eine Emulsion oder um ein reines Pflanzenöl handeln. Auch Duftstoffe sind in einer Ausführungsform der vorliegenden Erfindung als Wirkstoffe anzusehen.

Das zerkleinerte Pflanzenmaterial der Naturstoffmatrix 120 kann aus derselben Pflanze gewonnen wie das Extraktionsgut. Dabei können alle Teile der Pflanze verwendet werden, also z. B. die Blütenblätter, Blätter, Stängel oder Wurzeln als Ganzes, aber auch in gemahlener oder fein/grob geschnittener Form. So entsteht ein selbstklebendes Naturstoffpad 100 mit einer zu der physiologischen Wirkung korrespondierenden Optik und, durch einen dünnen und der Kontur der Naturstoffe folgenden Überzug 130, korrespondierenden Haptik.

Die Herstellung des Naturstoffpads 100 kann erfolgen, indem zunächst eine auf ihrer Haftseite mit einem Abziehpapier kaschierte Klebeschicht 110 bereitgestellt und mit nach unten gerichteter Haftschicht bzw. nach unten gerichtetem Abziehpapier auf einer Unterlage positioniert wird. Auf die Oberseite der so positionierten Klebeschicht kann dann zerkleinertes Pflanzenmaterial aufgelegt und so lange mit einem flüssigen Bindemittel besprüht werden, bis das Pflanzenmaterial teilweise oder vorzugsweise vollständig benetzt ist. Nach einer Trocknung des Bindemittels entstehen dadurch auf der Oberseite der Klebeschicht eine Naturstoffmatrix 120 und ein diese bedeckender Überzug 130, der aus dem Bindemittel besteht, aber als oberste Schicht keine Pflanzenteile mehr enthält. Das so erhaltene Schichtgebilde wird dann umgedreht und kann mit einer Stachelwalze behandelt werden, sodass die Klebeschicht 110 nebst assoziiertem Abziehpapier perforiert werden. Die Perforation reicht bis in die Naturstoffmatrix 120. Anschließend wird ein flüssiges Pflanzenextrakt auf die so perforierte Oberfläche des Schichtgebildes aufgetragen, sodass es im Verlauf einer anschließenden Einwirkzeit durch die Perforation in die Naturstoffmatrix 120 einsickern kann. In einem letzten Schritt wird das perforierte Abziehpapier abgezogen und durch ein neues, nicht perforiertes Abziehpapier 140 ersetzt. Das erhaltene Naturstoffpad 100 wird in Stücke geeigneter Größe zerschnitten und die Stücke werden einzeln luftdicht verpackt.

Das so erhaltene Naturstoffpad 100 kann vorzugsweise als Wirkstoffpflaster auf die Haut eines Benutzers geklebt werden. So können die Wirkstoffe, die aus Kräutern und anderen Naturprodukten gewonnen werden, räumlich begrenzt mit der Hautoberfläche in Kontakt treten und über die Haut aufgenommen werden. Die Wirkstoffe können eine medizinische, kosmetische, beruhigende, stimulierende oder therapeutische Wirkung haben. Beispielsweise können so in Wärmecremes zum Einsatz kommende natürliche Wirkstoffe in Pflasterform bereitgestellt und von einem Sportler oder einer Person mit rheumatischen Beschwerden verwendet werden. Auch als Nikotinpflaster kann das Naturstoffpad 100 zur Anwendung kommen. Als schmerzstillendes Pflaster auf Basis von beispielsweise Zahnkraut kann das Naturstoffpad 100 beispielsweise auf die Backe eines Benutzers geklebt werden.

In einer anderen Variante der Erfindung kann auch der Überzug 130 mit der Stachelwalze behandelt und perforiert werden, während die Klebeschicht 110 verschont bleibt. Die Wirkstoffabgabe erfolgt dann an der Oberseite des Naturstoffpads 100 durch den Überzug 130 hindurch, der in diesem Fall in der Herstellung noch mit einem selbstklebenden Schutzpapier überzogen werden kann, um eine Wirkstoffabgabe vor Aktivierung durch den Benutzer zu verhindern. In dieser Ausführungsvariante kann das Naturstoffpad 100 als Aufkleber für beispielsweise Mobiltelefone, Kleidungsstücke oder Möbel verwendet werden, das ätherische Öle einer Pflanze abgeben kann.

## Patentansprüche

1. Naturstoffpad (100) in Form eines Schichtsystems mit einer flächigen Naturstoffmatrix (120), die auf einem Träger (110) angeordnet und von einem Überzug (130) überdeckt ist, wobei die Naturstoffmatrix (120) ein Pflanzenmaterial, ein Bindemittel und am Pflanzenmaterial gehaltene oder anderweitig in der Naturstoffmatrix (120) verteilte Wirk- und/oder Duftstoffe umfasst,
**dadurch gekennzeichnet,**
**dass** das Pflanzenmaterial als Ganzes oder in geschnittener Form in der Naturstoffmatrix (120) vorliegt und der Überzug (130) an der Oberfläche nicht glatt ist, sondern den Konturen des Pflanzenmaterials in der Naturstoffmatrix (120) folgt.

2. Naturstoffpad nach Anspruch 1, **dadurch gekennzeichnet, dass** es sich bei dem Naturstoffpad (100) um ein selbstklebendes Naturstoffpad (100) handelt und der Träger (110) zumindest an seiner von der Naturstoffmatrix (120) abgewandten Unterseite eine Klebeoberfläche aufweist.

3. Naturstoffpad nach einem der vorhergehenden Ansprüche, **dadurch gekennzeichnet, dass** der Überzug (130) durch zusätzliches Bindemittel gebildet wird.

4. Naturstoffpad nach einem der vorhergehenden Ansprüche, **dadurch gekennzeichnet, dass** der Träger (110) und/oder der Überzug (130) eine Vielzahl an Öffnungen aufweisen, durch welche Wirk- und/oder Duftstoffe der Naturstoffmatrix (120) abgegeben werden können, wobei vorzugsweise vorgesehen ist, dass das Naturstoffpad (100) an der die Öffnungen aufweisenden Seite mit einem abziehbaren Schutzfilm (140) versehen ist.

5. Naturstoffpad nach einem der vorhergehenden Ansprüche, **dadurch gekennzeichnet, dass** das Bindemittel einen oder mehrere der folgenden Komponenten umfasst: Harnstoffleim, Naturharze, Methylzellulose, Stärke, Polylactide, Naturkautschuk, Polyvinylacetat, Polyurethan oder Cyanacrylat.

6. Naturstoffpad nach einem der vorhergehenden Ansprüche, **dadurch gekennzeichnet, dass** die Wirk- und/oder Duftstoffe pflanzlichen Ursprungs sind und vorzugsweise in flüssiger Form vorliegen, wobei die Flüssigkeit am Pflanzenmaterial oder einem zusätzlichen Trägermaterial innerhalb der Naturstoffmatrix (120) gebunden ist, wobei vorzugsweise vorgesehen ist, dass es sich bei dem zusätzlichen Trägermaterial um ein pulver- oder teilchenförmiges Materialwie beispielsweise Zeolithmaterial, oder um ein textiles Material handelt.

7. Naturstoffpad nach einem der vorhergehenden Ansprüche, **dadurch gekennzeichnet, dass** das Pflanzenmaterial zumindest teilweise aus Moosen oder Flechten gewonnen ist.

8. Naturstoffpad nach einem der Ansprüche 6 bis 7, **dadurch gekennzeichnet, dass** das Pflanzenmaterial und die Wirk- und/oder Duftstoffe zumindest teilweise auf dieselbe Pflanzenfamilie, vorzugsweise dieselbe Pflanzengattung und weiter vorzugsweise dieselbe Pflanzenart zurückgehen.

9. Naturstoffpad nach einem der vorhergehenden Ansprüche, **dadurch gekennzeichnet, dass** die Naturstoffmatrix (120) neben den Pflanzenmaterialien und dem Binder ferner Stoffe nicht biogenen Ursprungs enthält, beispielsweise Zeolithgranulat, Füllstoffe oder Speicherstoffe.

10. Verfahren zur Herstellung eines Naturstoffpads (100) nach einem der vorhergehenden Ansprüche mit den folgenden Schritten:
a) Bereitstellen des Trägers (110);
b) Positionieren des Trägers (110) auf einer Unterlage;
c) Auftragen des Pflanzenmaterials und des Bindemittels sowie gegebenenfalls eines zusätzlichen Trägermaterials für Wirk- und/oder Duftstoffe auf den Träger (110), um die Naturstoffmatrix (120) auszubilden; und
d) Ausbildung des Überzugs (130), vorzugsweise durch das Auftragen von weiterem Bindemittel auf die Naturstoffmatrix (120);
wobei die Wirk- und/oder Duftstoffe vor Ausbildung des Überzugs (130) oder nachträglich durch Öffnungen im Träger (110) oder Überzug (130) in die Naturstoffmatrix (120) eingebracht werden.

11. Verfahren nach Anspruch 10, **dadurch gekennzeichnet, dass** das Verfahren einen weiteren Schritt e) zur Perforierung des Trägers (110) oder des Überzugs (130) anhand vorzugsweise einer Stachelwalze aufweist, um Öffnungen in den Träger (110) oder Überzug (130) einzuarbeiten.

12. Verfahren nach einem der Ansprüche 10 bis 11, **dadurch gekennzeichnet, dass** der Träger (110) in Schritt a) mit einem abziehbaren Schutzfilm bereitgestellt wird und/oder dass ein abziehbarer Schutzfilm nach Schritt d) und vorzugsweise nach dem optionalen Schritt e) nachträglich auf den Träger (110) oder den Überzug (130) aufgebracht wird.

13. Verfahren nach einem der Ansprüche 10 bis 12, **dadurch gekennzeichnet, dass** die Auftragung von Bindemittel in Schritten c) und/oder d) durch Sprühen erfolgt.

14. Verwendung eines Naturstoffpads (100) nach einem der Ansprüche 2 bis 9 als Aufkleber für Oberflächen mobiler oder stationärer Gebrauchsgenstände.

15. Verwendung eines Naturstoffpads (100) nach einem der Ansprüche 1 bis 9 als Wirkstoff- oder Duftreservoir für Räume oder zur Anbringung an Kleidungsstücken.

## Claims

1. Natural material pad (100) in the form of a layer system with a flat natural material matrix (120), which is arranged on a carrier (110) and is covered by a coating (130), wherein the natural material matrix (120) comprises a plant material, a binder and active substances and/or fragrances held on the plant material or otherwise distributed in the natural material matrix (120),
**characterised in**
**that** the plant material is present as a whole or in cut form in the natural material matrix (120) and the coating (130) is not smooth on the surface but follows the contours of the plant material in the natural material matrix (120).

2. Natural material pad according to claim 1, **characterised in that** the natural material pad (100) is a self-adhesive natural material pad (100) and the carrier (110) has an adhesive surface at least on its underside facing away from the natural material matrix (120).

3. Natural material pad according to one of the preceding claims, **characterised in that** the coating (130) is formed by additional binder.

4. Natural material pad according to one of the preceding claims, **characterised in that** the carrier (110) and/or the coating (130) have a plurality of openings through which active substances and/or fragrances of the natural material matrix (120) can be released, it preferably being provided that the natural material pad (100) is provided with a peelable protective film (140) on the side having the openings.

5. Natural material pad according to one of the preceding claims, **characterised in that** the binder comprises one or more of the following components: Urea glue, natural resins, methyl cellulose, starch, polylactides, natural rubber, polyvinyl acetate, polyurethane or cyanoacrylate.

6. Natural material pad according to one of the preceding claims, **characterised in that** the active substances and/or fragrances are of plant origin and are preferably present in liquid form, the liquid being bound to the plant material or to an additional carrier material within the natural material matrix (120), it being preferably provided that the additional carrier material is a powdery or particulate material, such as zeolite material, or a textile material.

7. Natural material pad according to one of the preceding claims, **characterised in that** the plant material is at least partially obtained from mosses or lichens.

8. Natural material pad according to any one of claims 6 to 7, **characterised in that** the plant material and the active substances and/or fragrances are at least partly derived from the same plant family, preferably the same plant genus and further preferably the same plant species.

9. Natural material pad according to one of the preceding claims, **characterised in that** the natural material matrix (120), in addition to the plant materials and the binder, further contains substances of non-biogenic origin, for example zeolite granulate, fillers or storage substances.

10. Method of producing a natural material pad (100) according to one of the preceding claims, comprising the following steps:
a) providing the carrier (110);
b) positioning the carrier (110) on a support;
c) applying the plant material and the binder as well as optionally an additional carrier material for active substances and/or fragrances to the carrier (110) to form the natural material matrix (120); and
d) forming the coating (130), preferably by applying further binder to the natural material matrix (120);
wherein the active substances and/or fragrances are introduced into the natural material matrix (120) through openings in the carrier (110) or coating (130) prior to the formation of the coating (130) or subsequently.

11. Method according to claim 10, **characterised in that** the method comprises a further step e) for perforating the carrier (110) or the coating (130) by means of preferably a spiked roller in order to incorporate openings into the carrier (110) or coating (130).

12. Method according to one of claims 10 to 11, **characterised in that** the carrier (110) is provided with a peelable protective film in step a) and/or that a peelable protective film is subsequently applied to the carrier (110) or the coating (130) after step d) and preferably after the optional step e).

13. Method according to one of claims 10 to 12, **characterised in that** the application of binder in steps c) and/or d) is carried out by spraying.

14. Use of a natural material pad (100) according to one of claims 2 to 9 as a sticker for surfaces of mobile or stationary utensils.

15. Use of a natural material pad (100) according to one of claims 1 to 9 as an active substance or fragrance reservoir for rooms or for attachment to articles of clothing.

## Revendications

1. Coussinet en matière naturelle (100) sous la forme d'un système de couches avec une matrice de matière naturelle (120) plane, qui est disposée sur un support (110) et recouverte d'un revêtement (130), la matrice de matière naturelle (120) comprenant une matière végétale, un liant et des substances actives et/ou odorantes maintenues sur la matière végétale ou réparties d'une autre manière dans la matrice de matière naturelle (120),
**caractérisé en ce**
**que** la matière végétale est présente dans la matrice de matière naturelle (120) sous forme entière ou sous forme découpée et que le revêtement (130) n'est pas lisse à la surface, mais suit les contours de la matière végétale dans la matrice de matière naturelle (120).

2. Coussinet en matière naturelle selon la revendication 1, **caractérisé en ce que** le coussinet en matière naturelle (100) est un coussinet en matière naturelle autocollant (100) et **en ce que** le support (110) présente une surface adhésive au moins sur sa face inférieure opposée à la matrice en matière naturelle (120).

3. Coussinet en matière naturelle selon l'une des revendications précédentes, **caractérisé en ce que** le revêtement (130) est formé par un liant supplémentaire.

4. Coussinet en matière naturelle selon l'une des revendications précédentes, **caractérisé en ce que** le support (110) et/ou le revêtement (130) présentent un grand nombre d'ouvertures à travers lesquelles des substances actives et/ou odorantes de la matrice de matière naturelle (120) peuvent être libérées, sachant qu'il est de préférence prévu que le coussinet en matière naturelle (100) soit pourvu d'un film de protection (140) pelable sur la face présentant les ouvertures.

5. Coussinet en matière naturelle selon l'une des revendications précédentes, **caractérisé en ce que** le liant comprend un ou plusieurs des composants suivants : Colle d'urée, résines naturelles, méthylcellulose, amidon, polylactides, caoutchouc naturel, acétate de polyvinyle, polyuréthane ou cyanoacrylate.

6. Coussinet en matière naturelle selon l'une des revendications précédentes, **caractérisé en ce que** les substances actives et/ou odorantes sont d'origine végétale et se présentent de préférence sous forme liquide, le liquide étant lié à la matière végétale ou à une matière de support supplémentaire à l'intérieur de la matrice de matière naturelle (120), de préférence en prévoyant que la matière de support supplémentaire est une matière en poudre ou en particules, comme par exemple une matière zéolite, ou une matière textile.

7. Coussinet en matière naturelle selon l'une des revendications précédentes, **caractérisé en ce que** la matière végétale est obtenue au moins en partie à partir de mousses ou de lichens.

8. Coussinet en matière naturelle selon l'une des revendications 6 à 7, **caractérisé en ce que** la matière végétale et les substances actives et/ou odorantes proviennent au moins en partie de la même famille de plantes, de préférence du même genre de plantes et de préférence encore de la même espèce de plantes.

9. Coussinet en matière naturelle selon l'une des revendications précédentes, **caractérisé en ce que** la matrice de matière naturelle (120) contient, outre les matières végétales et le liant, des substances d'origine non biogène, par exemple des granulés de zéolithe, des charges ou des substances de stockage.

10. Procédé de fabrication d'un coussinet en matière naturelle (100) selon l'une des revendications précédentes, comprenant les étapes suivantes :
a) Préparation du support (110) ;
b) Positionnement du support (110) sur un soutien;
c) Application de la matière végétale et du liant, ainsi que, le cas échéant, d'une matière de support supplémentaire pour les substances actives et/ou odorantes, sur le support (110), afin de former la matrice de matière naturelle (120) ; et
d) Formation du revêtement (130), de préférence par l'application d'un autre liant sur la matrice de matière naturelle (120) ;
les substances actives et/ou odorantes étant introduites dans la matrice de matière naturelle (120) avant la formation du revêtement (130) ou ultérieurement par des ouvertures dans le support (110) ou le revêtement (130).

11. Procédé selon la revendication 10, **caractérisé en ce que** le procédé comprend une étape supplémentaire e) de perforation du support (110) ou du revêtement (130) à l'aide, de préférence, d'un rouleau à picots, afin de pratiquer des ouvertures dans le support (110) ou le revêtement (130).

12. Procédé selon l'une des revendications 10 à 11, **caractérisé en ce que** le support (110) est fourni à l'étape a) avec un film protecteur pelable et/ou **en ce qu'**un film protecteur pelable est appliqué ultérieurement sur le support (110) ou le revêtement (130) après l'étape d) et de préférence après l'étape e) optionnelle.

13. Procédé selon l'une des revendications 10 à 12, **caractérisé en ce que** l'application de liant aux étapes c) et/ou d) est réalisée par pulvérisation.

14. Utilisation d'un coussinet en matière naturelle (100) selon l'une des revendications 2 à 9 comme autocollant pour des surfaces d'objets utilitaires mobiles ou stationnaires.

15. Utilisation d'un coussinet en matière naturelle (100) selon l'une des revendications 1 à 9 comme réservoir de substance active ou de parfum pour des pièces ou pour l'application sur des vêtements.
